# EUROPEAN PATENT APPLICATION

(11) **EP 4 529 870 A1**
(43) Date of publication of application: **02.04.2025**
(21) Application number: 24202310.9
(22) Date of filing: 24.09.2024
(51) Int. Cl.: A61B 17/86

(54) **PEDICLE SCREW TIP INSERTION FEATURE**

(30) Priority: 28.09.2023 US 202363541080 P
(71) Applicant: K2M, Inc, Leesburg, VA 20175 (US)
(72) Inventor: Genovese, Daniel, Great Falls, 22066 (US); Lang, Brittany, Warrenton, 20187 (US)
(74) Representative: Keller Schneider Patentanwaltsgesellschaft mbH

(57) **Abstract**

A bone screw having cutting features to facilitate the insertion thereof. The bone screw including a shaft portion having a terminal end and a first thread terminating at the terminal end. The first thread including a first crest and a first cut that extends along a helix from the terminal end such that the first crest is divided to create a cutting edge along the first cut.

## Description

### BACKGROUND

Bone screws are often utilized as part of an internal fixation device used to affix and align bones. For instance, bone screws are utilized with bone plates and rods to repair the fracture or deformity of a bone structure. While bone screws come in all shapes and sizes and have a wide variety of applications, all bone screws are designed to be inserted and threaded into bone tissue. This is done by simultaneously applying an axial force and torque that cause the bone screw to cut and thread into bone tissue until the screw is fully inserted. The amount of axial force and torque needed to insert any given bone screw will depend on various factors including the design of the thread and tip of the screw, which may be designed for placement after drilling or other preparation of the bone or themselves may be designed to facilitate cutting and threading.

Bone screws are often inserted into bone that may be damaged or located at an intricate part of the body. In many such instances, it is desired that the insertion of a bone screw is done quickly and/or with minimal force. For example, reducing the axial force needed to insert bone screws can reduce stress on and movement of the body which can improve navigation accuracy and reduce imaging. Therefore, advancements in cutting features that improve the efficiency of bone screw insertion is desired.

### BRIEF SUMMARY

Some aspects herein relate to a bone screw that includes cutting features located at the tip of the bone screw and along the thread(s). The tip of the bone screw defines an initial contact surface located at the distal end of the screw. The contact surface may have a plurality of sharp corners and edges created by grooves, notches, and/or slots carved therein and thereby creating cutting features at the tip of the bone screw. The sharp edges and corners are designed to catch and cut bone tissue to help facilitate the insertion of the bone screw. Some of these cutting features may run along the crest and/or root of the thread of the bone screw such that the crest and/or root are split into two sides. Other cutting features are carved into the distal end (i.e., the contact surface) and extend into a hollow portion located within the bone screw, while other cutting features remain external to the hollow portion. Yet some of the cutting features extend along a helical path similar to that of the thread or are extending in a direction transverse the path of the thread. Most of the cutting features are located near the distal end of the bone screw where the depth of the thread is tapered until it terminates.

In another aspect, a bone screw may include a shaft portion having a terminal end and a first thread terminating at the terminal end. The first thread may include a first crest and a first cut that extends along the helix of the first crest from the terminal end such that the first crest is divided along the first cut. In such arrangements, the bone screw may include a second thread having a second crest disposed along the shaft portion and terminating at the terminal end such that the bone screw is a dual-lead threaded screw. In some such arrangements, the second crest may have a second cut that extends along the helix of the second crest from the terminal end such that the second crest is divided along the second cut. In such arrangements, the shaft portion may include an aperture extending along its length and having a cylindrical sidewall at the terminal end. The aperture may define a plane that is perpendicular to the longitudinal axis of the bone screw. In some such arrangements, the first and second cuts may extend through the sidewall into the aperture. In other arrangements, the first and second cuts may be above the sidewall.

In other arrangements, a portion of the first and second cuts may extend through the sidewall into the aperture. A height of the first crest may taper as the first thread approaches the terminal end of the shaft portion, and the first cut may have a varying depth as the first crest tapers. The first cut may extend only partially along the shaft portion. A portion of the first cut may extend into the first thread and the shaft portion to a depth beyond a root of the first thread. The first cut may extend from the terminal end for a length of 2.7 mm. The first crest may include a notch on at least one side of the first cut. The sidewall may include a nick or partial cut at the terminal end.

In accordance with another aspect, a bone screw may include a head portion, a shaft portion, and a tip portion. The shaft portion may be threaded with a first thread having a first root and a second thread having a second root. The head portion may be attached to the shaft portion at a proximal end. The tip portion may be disposed at the distal end of the shaft. The tip portion may define a terminal end where the first and second threads terminate. The first root may have a first cut that extends along a helix of the first root from the terminal end such that the first root is divided along the first cut. The second root may have a second cut that extends along a helix of the second root from the terminal end such that the second root is divided along the second cut. In some such arrangements, a first crest of the first thread may have a third cut that extends along the helix of the first crest from the terminal end such that the first crest is divided along the third cut. The second crest of the second thread may have a fourth cut that extends along the helix of the second crest from the terminal end such that the second crest is divided along the fourth cut.

In other arrangements, the angle of the tapered tip of a bone screw may vary from the taper angle of a helical cut defining a groove splitting a thread crest. For example, the angle of the tapered tip and the taper angle of the helical cut may have the same angle, e.g., 30 degrees, which results in the helical cut terminating or ending before reaching a point along the maximum outer diameter of the bone screw. Alternatively, the taper angle of the helical cut may taper outward at a less sharp angle (i.e., smaller angle) which results in the helical cut extending along the maximum outer diameter of the bone screw shank. In other embodiments, the taper angle of the helical cut groove splitting thread crests may taper outward at a sharper angle (i.e., greater angle) than that of the tapered tip of the bone screw, which results in the helical cut terminating or ending closer to the distal end of the bone screw. In this manner, a length of a helical cut is determined from the angle by which the helical cut and the tapered tip of the bone screw extend outward from the shaft of the bone screw, respectively.

In another arrangement, the tip portion includes an aperture having a cylindrical sidewall at the terminal end. The aperture may define a plane that is perpendicular to the longitudinal axis of the bone screw. In such arrangements, the first and second cuts may extend through the sidewall into the aperture. The first and second cuts may be above the sidewall. A segment of the first and second cuts may extend through the sidewall into the aperture. The tip portion may taper between the distal end of the shaft and the terminal end of the tip portion. Each of the first and second cuts may extend from the terminal end for a length of approximately 2.7 mm. The head portion may include a hexalobe socket. The bone screw may be a pedicle screw made from biocompatible material.

In accordance with another aspect, a bone screw may be inserted into bone by a process. In the process, a tip of the bone screw may be positioned against a bone, the tip having a cutting feature including at least a first cut splitting a first crest of a first thread and a first notch at the tip. Next, a portion of the first cut may be pushed against the bone. Finally, the bone screw may be inserted into the bone by applying an axial force and a torsional force to the bone screw. In such arrangements, the axial force may be a lower axial force compared to that which normally is applied under similar circumstances. In other arrangements, a tool may be inserted into a head of the bone screw before pushing a portion of the first cut against the bone. The tool may have an end portion that corresponds to a cavity in the head of the bone screw. The insertion of the bone screw may include bone that is cut into with the cutting feature of the tip. The bone screw may be fully inserted into bone with less torque than a similar screw without the first cut and the first notch when an equivalent axial force is applied to both. In other arrangements, less axial force may be applied to fully insert the bone screw into bone compared to that which is required to insert a similar screw without the first cut and first notch.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1 is a side view of a screw in accordance with an embodiment of the present disclosure;
FIG. 2 is a cross sectional side view of the screw of FIG. 1 taken along line A-A;
FIG. 3 is a side view of the screw of FIG. 1;
FIG. 4 is a perspective view of the screw tip of the screw of FIG. 1;
FIG. 5 is a side view of the screw tip of the screw of FIG. 1;
FIG. 6 is a bottom view of a screw tip of a screw in accordance with an embodiment of the present disclosure;
FIG. 7 is a perspective view of the screw tip of FIG. 6;
FIG. 8 is a top view of the screw of FIG. 1;
FIG. 9 is a perspective view of the screw tip of a screw in accordance with an embodiment of the present disclosure;
FIG. 10 is side view of a screw tip of a screw in accordance with an embodiment of the present disclosure; and
FIG. 11 is a side view of a screw tip of a screw in accordance with an embodiment of the present disclosure.

### DETAILED DESCRIPTION

As used herein unless stated otherwise, the term "anterior" means toward the front part of the body, and the term "posterior" means toward the back part of the body. When referring to specific directions in the following discussion of a certain device, the terms "proximal" and "distal" are to be understood in regard to the device's orientation and position during exemplary application to human body. Thus, the term "proximal" means closer to the operator or in a direction toward the operator, and the term "distal" means more distant from the operator or in a direction away from the operator. In addition, the terms "about," "generally," and "substantially" are intended to mean that deviations from absolute are included within the scope of the term so modified.

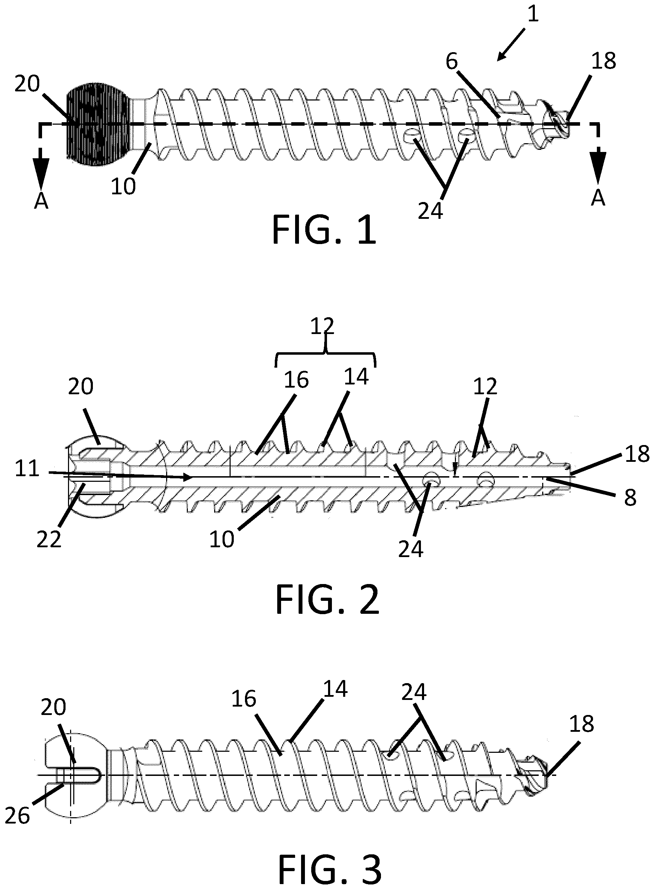

In a first aspect, the present disclosure relates to a bone screw with cutting features, particularly cutting features at the tip of the bone screw to facilitate insertion of the bone screw into bone tissue. The bone screw may be suited for a particular purpose, such as a pedicle screw used for spinal fusion. One embodiment, as shown in FIGS. 1-3, the bone screw includes shaft 10 having thread 12 disposed thereon in a helical configuration and head 20 located at opposite end from distal end 18. Shaft 10 further includes hollow portion 8 defining an aperture 11 that extends the full length of the shaft along the central axis of the shaft, the diameter of the hollow portion being constant throughout. In alternative embodiments, hollow portion 8 may only extend partially along the central axis of shaft 10 or the shaft could be completely solid. Additionally, shaft 10 includes one or more shaft holes or fenestrations 24 that are disposed along the length of shaft 10 between portions of crest 14. Shaft holes extending through shaft 10 and into hollow portion 8 such that the shaft holes are in communication with the hollow portion. The inclusion of hollow portion 8 and holes 24 permit the screw to be utilized to deliver bone cement, saline, or other materials to the bone, depending on surgeon preference and indication. In other embodiments, shaft 10 may have no shaft holes. For instance, there would be no need for holes in the case of a solid shaft 10. Moreover, screws in other embodiments can still include hollow portion 8 defining an aperture 11, but no holes 24. Hollow portion 8 can be sized and shaped to receive a K-wire or other similar structure to aid in the guidance of the screw to the surgical site.

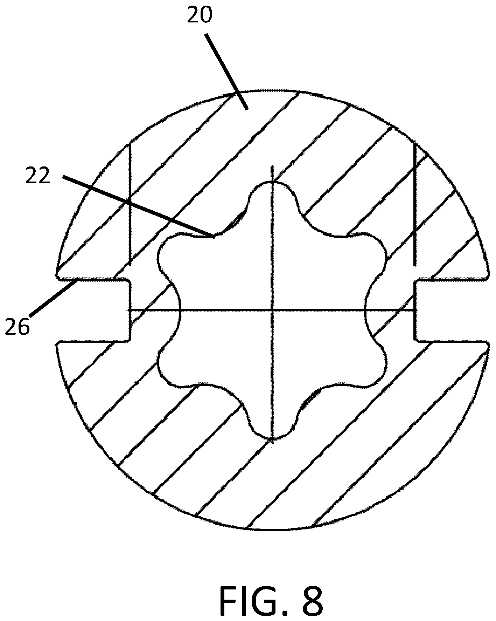

Continuing with FIGS. 1-3, head 20 extends proximally from shaft 10, the head having a curved exterior. Head 20 includes cavity 22 that defines a hexalobe recess and side slots 26 extending into a portion of the outer wall of the head on opposite sides of the head, as shown in FIG. 8, one or both of which are designed to receive a tool for applying axial force to shaft 10 via the head. In some embodiments, cavity 22 may be in communication with hollow portion 8. The bone screw may be fabricated from various biocompatible materials, for example, but not limited to: titanium, titanium-alloys, stainless steel, etc.

Thread 12 includes crest 14 and root 16. Crest 14 extends away from and radially around shaft 10, and Root 16 wraps around adjacent to the shaft. The distance that crest 14 extends away from root 16 defines the depth of thread 12, which is generally constant along shaft 10 but does gradually decrease as the thread approaches a terminal end opposite head 20. In other words, the depth of the thread begins to taper a short distance away from distal end 18 until the thread terminates at the distal end. This tapered portion of thread 12 coincides with the taper portion of shaft 10 located at the distal end. Thread 12 has one or more flutes, cuts, slits, or notches disposed thereon, such as side cut 6. In other embodiments, the bone screw has additional cutting features described more fully below to facilitate insertion of shaft 10 into bone.

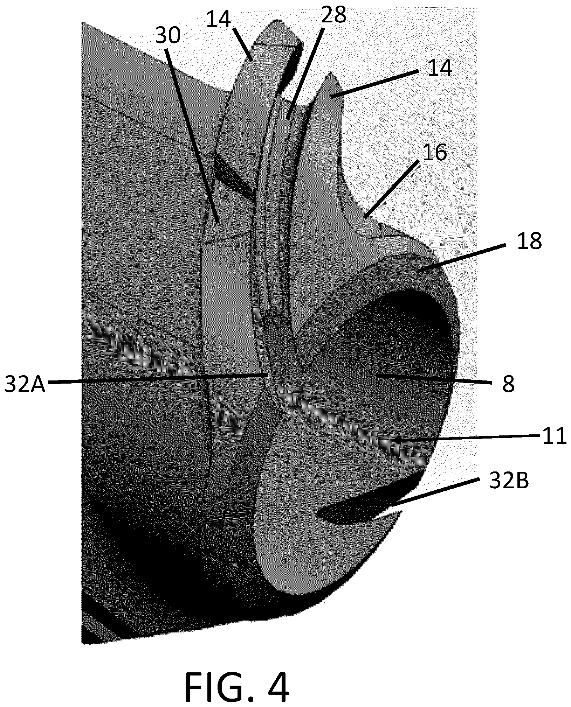

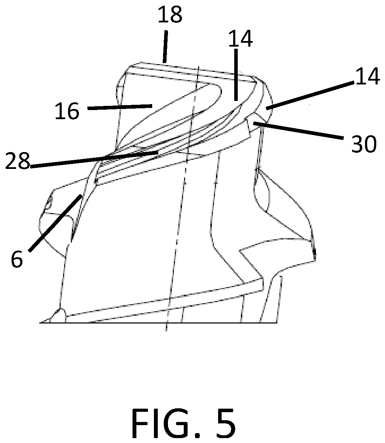

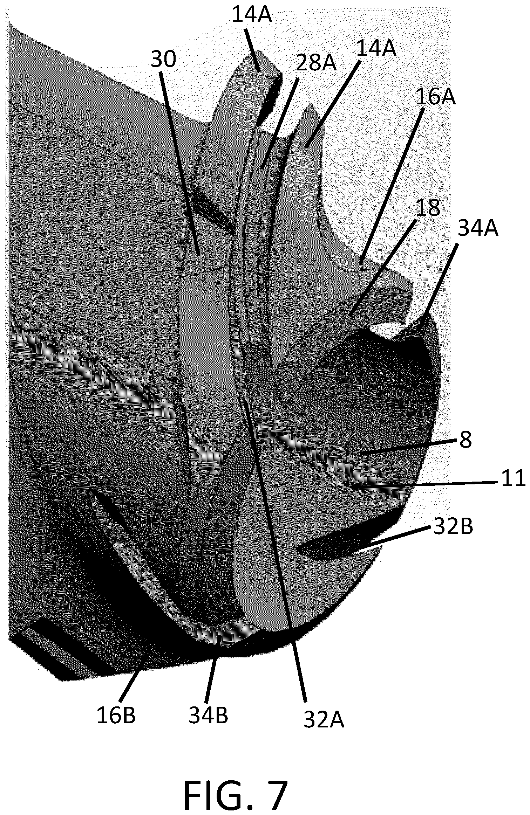

In reference to FIGS 4-9, crest 14 includes first cut 28 that tracks along the helical path of the crest such that the crest is split into two sides. Although shown as splitting the crest equally in the depicted embodiment, crest 14 may be equally or unequal split into two sides in other embodiments. First cut 28 extends along crest 14 at least until a break (i.e., side cut 6) in the crest, as shown in FIG. 5. Alternatively, first cut 28 may be designed to split crest 14 along the tapered portion of thread 12 starting from distal end 18 of shaft 10.

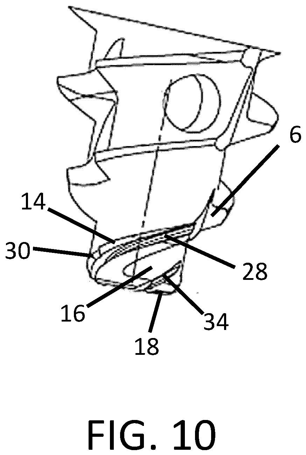

Crest 14 also includes groove 30 that is disposed on one or both sides of crest 14 near the distal end of shaft 10. The depth of first cut 28 extends through thread 12 and may extend to a depth beyond root 16 (i.e., the minor diameter of thread 12) but generally remains above or external to hollow portion 8 of shaft 10. In other words, a portion of first cut 28 may reach a depth that is closer to the longitudinal axis of shaft 10 than root 16. A majority of first cut 28 does not penetrate completely through shaft 10 and into hollow portion 8. However, first cut 28 includes first notch 32A that pierces into hollow portion 8 which creates a nick or slot-like feature at distal end 18. First notch 32A extends along first cut 28 for an initial length that is shorter than the full length of the first cut, as shown in FIG. 4. For example, first notch 32A may extend a length that is around 2-5 mm, but ideally the length should terminate after approximately 2.7 mm. First cut 28 may be terminated abruptly by another cutting feature carved into crest 14 (as shown in FIG. 10) or gradually with the depth of the first cut tapering out. Additionally, distal end 18 of shaft 10 includes second notch 32B that cuts through shaft 10 and into hollow portion 8 and extends along the shaft in a direction diagonal to the surface of the distal end. Second notch 32B may or may not be associated with a second thread that is disposed along shaft 10 similar to first notch 32A. In some embodiments, shaft 10 may include only first notch 32A or second notch 32B at distal end 18.

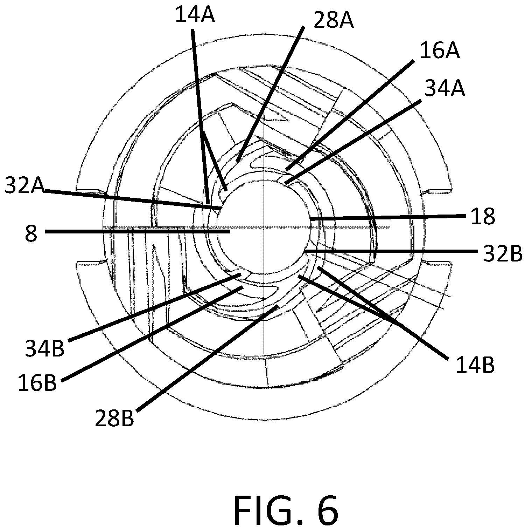

Shaft 10 may be single threaded, dual-lead threaded, and/or dual-quad lead threaded. When a bone screw has multiple threads, the crest of each thread may cut and notched similar to thread 12 containing first cut 28 and first notch 32A. For example, as shown in Fig. 6, first crest 14A and second crest 14B have first cut 28A and second cut 28B along with first notch 32A and second notch 32B, respectively. Adjacent to first crest 14A and second crest 14B is first root 16A and second root 16B that include first root notch 34A and second root notch 34B, respectively. First and second root notches 34A, 34B extend along the helical path of first and second root 16A, 16B, respectively, at a similar length as first notch 32A. In some embodiments, however, the length of first and second notches 32A, 32B and first and second root notches 34A, 34B may be substantially equal or unequal. When a bone screw has multiple threads, each root and crest of the various threads may include a notch feature at the distal end of the screw. For example, as shown FIGS. 6-7, distal end 18 includes, among other features, first notch 32A, second notch 32B, first root notch 34A, and second root notch 32B. In this manner, cutting features are disposed at the tip of shaft 10 to facilitate insertion of the shaft into bone tissue by enhancing the ability of the tip to cut and start threading into the bone.

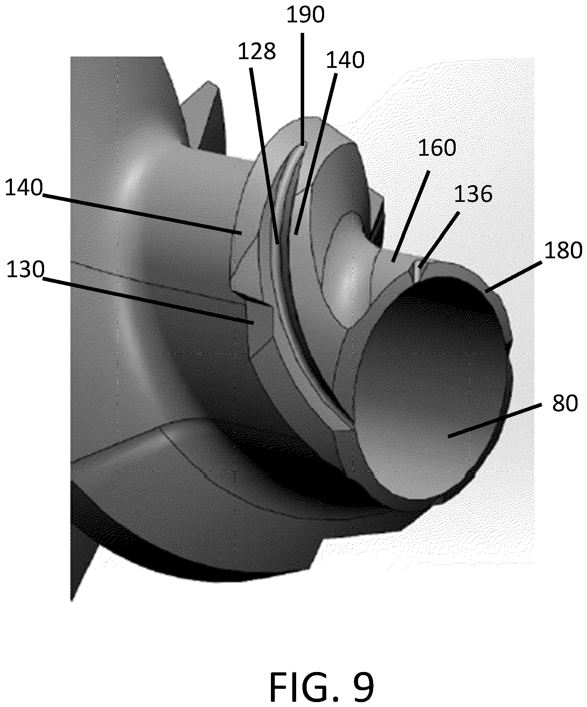

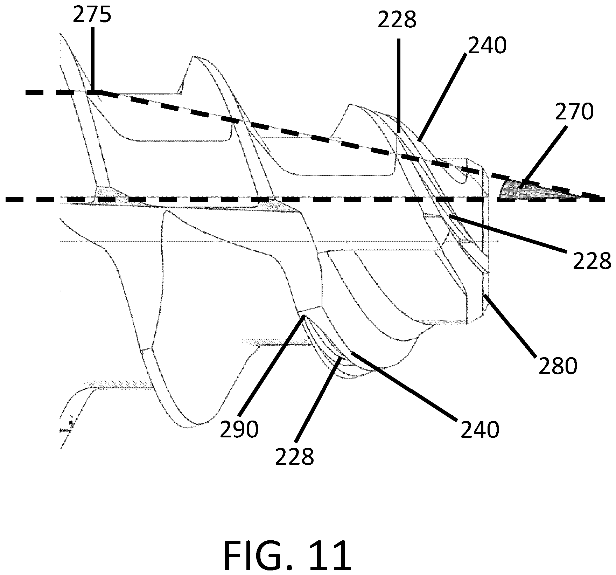

In another embodiments, the thread of the bone screw may include cutting features that do not extend into the hollow portion of the shaft. For example, as shown in FIG. 9, root 160 includes first root cut 136 that extends along the helix of the root creating a groove-like feature at distal end 180 that remains outside or above hollow portion 80. In some embodiments, there may be numerous cutting features similar to first root cut 136 that are disposed along distal tip 180. Additionally, crest 140 includes first cut 128 that splits the crest but does not include a notch feature at distal end that extends into hollow portion 80. First cut 128 extends along a tapered portion of the bone screw and gradually fades as the depth of the first cut diminishes until it reaches a terminal end 190. As shown in FIGS. 9 and 11, first cut 128, 228 ends at terminal end 190, 290 along the tapered tip of the bone screw and never reaches a point 275 along the maximum diameter of the bone screw. In such instances, the angle 270 of the tapered tip (i.e., the angle by which the tapered tip extends outward from the shank of the bone screw) is less than or equal to the angle or tapered angle of first cut 128, 228 (i.e., the angle by which the helical cut extends outward from the shank of the bone screw). Accordingly, in instances where the angle of a tapered tip (similar to angle 270) of a bone screw is less than the angle or tapered angle of a helical cut (similar to first cut 128 or 228), the helical cut terminates closer to the distal end of the bone screw. Alternatively, in instances where an angle of the tapered tip is greater than the angle or tapered angle of a helical cut, the helical cut extends along a crest past a point where the bone screw has reached its maximum diameter. In other words, the smaller the angle by which a helical cut extends outward from a shank of a bone screw in comparison to the angle by which a tapered tip or thread extends outward from the shank of the bone screw, the deeper and longer the helical cut will extend along the thread splitting the crest thereof. In some instances, the angle of a tapered tip (e.g., angle 270) may range from 15 degrees to 60 degrees and the angle of the helical cut or helical groove (e.g., first cut 128 or 228) may range from 15 degrees to 60 degrees. It is further contemplated that the bone screws described herein may be non-cannulated screws.

In another aspect, the present disclosure relates to kit that may include bone screws in a single package or in multiple packages that may be selected as needed by medical personnel. Such kits may include pedicle screws configured to be used for spinal fusion, the pedicle screws including insertion features, such as first cut 28A, first notch 32A, first root cut 136, and/or first root notch 34A. In some embodiments, the kit may include dual-lead and/or dual-quad lead threaded screws that have split threads and notch-like features at the distal end of the screws.

In yet another aspect, the present disclosure relates to a method of inserting bone screws into bone tissues. The method may include positioning a tip of a bone screw against bone, the tip including various cutting features described above such as first cut 28A, first notch 32A, first root cut 136, and/or first root notch 34A. Once the tip is positioned, inserting the head of tool into the head of the bone screw that corresponds to a cavity in the head of the bone screw. Once the tool is inserted, pushing a portion of the cutting features against the bone and inserting the screw into the bone by simultaneously applying an axial force and torque to the shaft of the screw, the axial force being a lower axial force compared to the force that would be applied to a similar bone screw without the cutting features described herein. Alternatively, once the cutting features are pressed against the bone, inserting the bone screw with less rotations than a bone screw without such cutting features when an equivalent axial force is applied. The cutting features described herein were tested to confirm these results.

It is to be understood that the disclosure set forth herein includes any possible combinations of the particular features set forth above, whether specifically disclosed herein or not. For example, where a particular feature is disclosed in the context of a particular aspect, arrangement, configuration, or arrangement, that feature may also be used, to the extent possible, in combination with and/or in the context of other particular aspects, arrangements, configurations, and arrangements of the technology, and in the technology generally.

Furthermore, although the technology herein has been described with reference to particular features, it is to be understood that these features are merely illustrative of the principles and applications of the present technology. It is therefore to be understood that numerous modifications, including changes in the sizes of the various features described herein, may be made to the illustrative arrangements and that other arrangements may be devised without departing from the spirit and scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

Preferred aspects of the invention are given below:
1. A bone screw, comprising,
   a shaft portion having a terminal end and a first thread terminating at the terminal end, the first thread including a first crest and a first cut that extends along a helix of the first crest from the terminal end such that the first crest is divided along the first cut.
2. The bone screw of aspect 1, further comprising a second thread having a second crest disposed along the shaft portion and terminating at the terminal end such that the bone screw is a dual-lead threaded screw.
3. The bone screw of aspect 2, wherein the second crest has a second cut that extends along a helix of the second crest from the terminal end such that the second crest is divided along the second cut.
4. The bone screw of aspect 3, wherein the shaft portion includes an aperture extending along its length and having a cylindrical sidewall at the terminal end, the aperture defining a plane that is perpendicular to a longitudinal axis of the bone screw.
5. The bone screw of aspect 4, wherein the first and second cuts extend through the sidewall into the aperture.
6. The bone screw of aspect 4, wherein the first and second cuts are above the sidewall.
7. The bone screw of aspect 5, wherein a portion of the first and second cuts extend through the sidewall into the aperture.
8. The bone screw of aspect 1, wherein a height of the first crest tapers as the first thread approaches the terminal end of the shaft portion, the first cut having a varying depth as the first crest tapers.
9. The bone screw of aspect 1, wherein the first cut extends only partially along the shaft portion.
10. The bone screw of aspect 1, wherein a portion of the first cut extends into the first thread and the shaft portion to a depth beyond a root of the first thread.
11. The bone screw of aspect 8, wherein the first cut extends from the terminal end for a length of 2.7 mm.
12. The bone screw of aspect 1, wherein the first crest includes a notch on at least one side of the first cut.
13. The bone screw of aspect 1, wherein the sidewall includes a nick at the terminal end.
14. A bone screw, comprising,
   a head portion;
   a shaft portion threaded with a first thread having a first root and a second thread having a second root, the head portion attached to the shaft portion at a proximal end; and
   a tip portion disposed at a distal end of the shaft, the tip portion defining a terminal end where the first and second threads terminate,
   wherein the first root has a first cut that extends along a helix of the first root from the terminal end such that the first root is divided along the first cut, and the second root has a second cut that extends along a helix of the second root from the terminal end such that the second root is divided along the second cut.
15. The bone screw of aspect 14, wherein a first crest of the first thread has a third cut that extends along a helix of the first crest from the terminal end such that the first crest is divided along the third cut, and a second crest of the second thread has a fourth cut that extends along a helix of the second crest from the terminal end such that the second crest is divided along the fourth cut.
16. The bone screw of aspect 14, wherein the tip portion includes an aperture having a cylindrical sidewall at the terminal end, the aperture defining a plane that is perpendicular to a longitudinal axis of the bone screw.
17. The bone screw of aspect 16, wherein the first and second cuts extend through the sidewall into the aperture.
18. The bone screw of aspect 16, wherein the first and second cuts are above the sidewall.
19. The bone screw of aspect 16, wherein a segment of the first and second cuts extend through the sidewall into the aperture.
20. The bone screw of aspect 14, wherein the tip portion tapers between the distal end of the shaft and the terminal end of the tip portion.
21. The bone screw of aspect 14, wherein each of the first and second cuts extend from the terminal end for a length of approximately 2.7 mm.
22. The bone screw of aspect 14, wherein the head portion includes a hexalobe socket.
23. The bone screw of aspect 14, wherein the bone screw is a pedicle screw made from biocompatible material.
24. A method of inserting a bone screw, comprising:
   positioning a tip of the bone screw against a bone, the tip having a cutting feature including at least a first cut splitting a first crest of a first thread and a first notch at the tip;
   pushing a portion of the first cut against the bone; and
   inserting the bone screw into the bone by applying an axial force and torsional force to the bone screw.
25. The method of aspect 24, wherein the axial force is a lower axial force compared to that which normally is applied under similar circumstances.
26. The method of aspect 24, further comprising inserting a tool into a head of the bone screw before pushing a portion of the first cut against the bone.
27. The method of aspect 26, wherein the tool has an end portion that corresponds to a cavity in the head of the bone screw.
28. The method of aspect 24, wherein the inserting of the bone screw includes cutting into the bone with the cutting feature of the tip.
29. The method of aspect 24, wherein the bone screw is fully inserted into bone with less torque than a similar screw without the first cut and the first notch when an equivalent axial force is applied to both.
30. The method of aspect 24, wherein less axial force is applied to fully insert the bone screw into bone compared to that which is required to insert a similar screw without the first cut and first notch.

## Claims

1. A bone screw, comprising,
a shaft portion having a terminal end and a first thread terminating at the terminal end, the first thread including a first crest and a first cut that extends partially along a helix of the first crest from the terminal end such that the first crest is divided along the first cut.

2. The bone screw of claim 1, further comprising a second thread having a second crest disposed along the shaft portion and terminating at the terminal end such that the bone screw is a dual-lead threaded screw, and wherein the first thread further defines a first root and a second cut that extends partially along a helix of the first root from the terminal end.

3. The bone screw of claim 2, wherein the second crest has a third cut that extends along a helix of the second crest from the terminal end such that the second crest is divided along the third cut.

4. The bone screw of any one of claims 2 or 3, further comprising a second root of the second thread having a fourth cut that extends along a helix of the second root.

5. The bone screw of any one of claims 1 to 4, wherein the shaft portion includes an aperture extending along its length and having a cylindrical sidewall at the terminal end, the aperture defining a plane that is perpendicular to a longitudinal axis of the bone screw.

6. The bone screw of claim 5, wherein at least one of the first, the second and the third cuts extend through the sidewall into the aperture.

7. The bone screw of any one of claims 5 or 6, wherein at least one of the first and the third cuts does not extend through the sidewall.

8. The bone screw of any one of claims 5 to 7, wherein a portion of at least one of the first and the third cuts extend through the sidewall into the aperture.

9. The bone screw of any one of claims 1 to 8, wherein a height of the first crest tapers as the first thread approaches the terminal end of the shaft portion, the first cut having a varying depth as the first crest tapers.

10. The bone screw of any one of claims 2 to 9, wherein a portion of the first cut extends into the first thread and the shaft portion to a depth beyond the first root of the first thread.

11. The bone screw of any one of claims 1 to 10, wherein the first cut extends from the terminal end for a length of 2.7 mm.

12. The bone screw of any one of claims 1 to 11, wherein the first crest includes a notch on at least one side of the first cut.

13. The bone screw of any one of claims 5 to 12, wherein the sidewall includes a notch at the terminal end.

14. The bone screw of any one of claims 1 to 13, further comprising a tip segment that tapers towards the terminal end.

15. The bone screw of any one of claims 1 to 14, wherein the shaft portion is a cannulated shaft having a hollow center.
